# EUROPÄISCHE PATENTSCHRIFT

(11) **EP 0 629 376 B1**
(45) Veröffentlichungstag und Bekanntmachung des Hinweises auf die Patenterteilung: **15.10.1997**
(21) Anmeldenummer: 94107381.9
(22) Anmeldetag: 11.05.1994
(51) Int. Cl.: A61B 6/00

(54) **Fahrbares Röntgengerät**
Mobile X-ray apparatus
Appareil mobile à rayons X

(30) Priorität: 27.05.1993 DE 4317713
(43) Veröffentlichungstag der Anmeldung: 21.12.1994
(73) Patentinhaber: SIEMENS AKTIENGESELLSCHAFT, 80333 München (DE)
(72) Erfinder: Exner, Wolfgang, Dr., D-01139 Dresden (DE)

(56) Entgegenhaltungen:
- EP-A- 0 231 969
- EP-A- 0 293 228
- EP-A- 0 554 711
- DE-U- 7 525 125
- DE-U- 8 521 246
- DE-U- 8 709 994

## Beschreibung

Es sind fahrbare Röntgengeräte bekannt, die auf einem Fahrgestell ein Bedienpult, ein Stativ und einen am Stativ gelagerten Röntgenstrahler aufweisen. Das Stativ mit dem Röntgenstrahler ist dabei relativ zum Fahrgestell um eine vertikale Achse schwenkbar, damit das Röntgengerät gut positionierbar und manövrierbar ist. Hierzu ist das Stativ mit dem Fahrgestell schwenkbar verbunden (DE-OS 2 212 510, US-PS 5 067 145). Diese Lösung bringt Probleme hinsichtlich der Kippsicherheit aufgrund der Schwerpunktverlagerung und erfordert zusätzliche Maßnahmen im Bereich der Fußbaugruppen.

In DE-U-85 21 246 ist ein ähnliches fahrbares Röntgengerät beschrieben, bei dem mit dem Stativ ein Bedienpult schwenkbar verbunden ist. Die einzelnen, mit dem Fahrgestell verbundenen Komponenten sind individuell einstellbar, so daß eine optimale Gewichtsverteilung nicht immer gewährleistet ist.

Der Erfindung liegt die Aufgabe zugrunde, ein Röntgengerät der eingangs genannten Art so auszubilden, daß unabhängig vom jeweiligen Schwenkwinkel des Röntgenstrahlers immer etwa gleiche Gewichtsverteilungen herrschen.

Diese Aufgabe ist erfindungsgemäß gelöst durch die Merkmale des Patentanspruches 1. Der Vorteil der erfindungsgemäßen Lösung besteht darin, daß das Bedienpult mit den darin angeordneten Komponenten permanent als Gegengewicht zum Röntgenstrahler wirkt. Dies ist bei der Lösung gemäß der US-PS 5 067 145 nicht der Fall, bei der nur der Röntgenstrahler relativ zum Fahrgestell um eine vertikale Achse verschwenkbar ist. Durch die Erfindung ist es möglich, das Fahrgestell parallel neben einem Bett aufzustellen. Ein Unterfahren des Bettes, das beim Stand der Technik wegen der erforderlichen Standsicherheit erfolgen muß, kann entfallen.

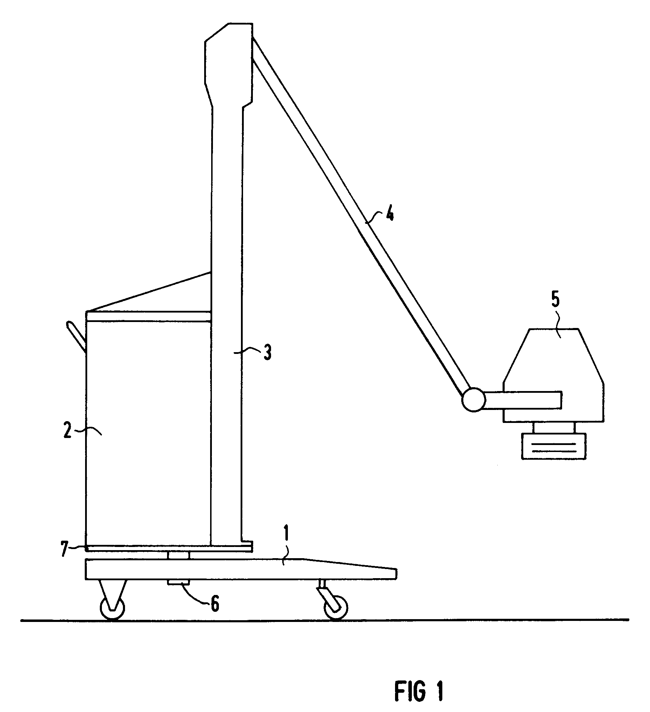

Die Erfindung ist nachfolgend anhand eines in der Zeichnung in drei verschiedenen Ansichten in den FIG 1 bis 3 dargestellten Ausführungsbeispiels näher erläutert.

In der Zeichnung ist ein Fahrgestell 1 dargestellt, das auf dem Boden eines Raumes verfahrbar ist und ein Bedienpult 2 sowie ein Stativ 3 trägt. Ein Röntgenstrahler 5 ist mit dem Stativ 3 über einen Schwenkarm 4 verbunden.

Das Bedienpult 2 und das Stativ 3 sind auf einer Montagegrundplatte 7 befestigt, welche um eine vertikale Achse 6 schwenkbar mit dem Fahrgestell 1 verbunden ist.

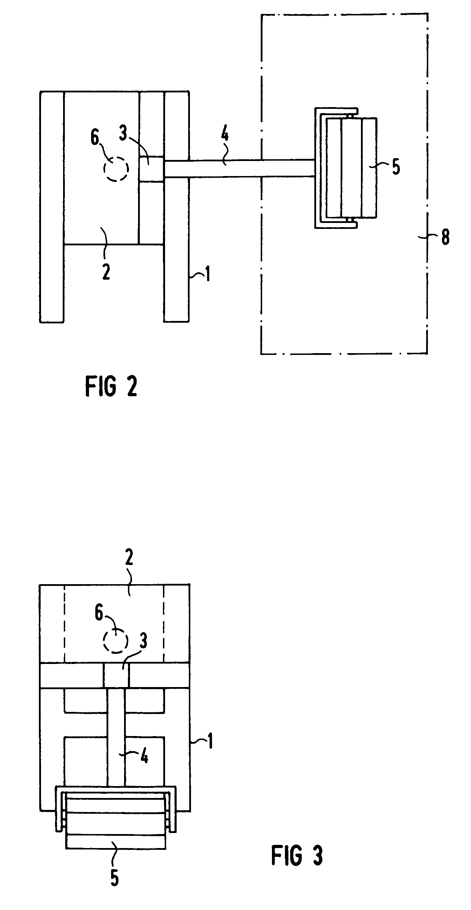

Die FIG 2 zeigt das Röntgengerät gemäß FIG 1 in der Arbeitsstellung. Das Fahrgestell 1 ist parallel zu einem Bett 8 ausgerichtet und der Röntgenstrahler 5 ist um die Achse 6 um 90° gegenüber der FIG 1 geschwenkt, so daß er über dem auf dem Bett 8 liegenden Patienten liegt. Ein Unterfahren des Bettes 8 mit dem Fahrgestell 1 ist nicht erforderlich, da das Bedienpult 2 ein Gegengewicht zum Röntgenstrahler 5 darstellt.

Die FIG 3 zeigt alle Komponenten des Röntgengerätes in Parkstellung, in der der Arm 4 mit dem Röntgenstrahler 5 parallel zu den beiden Auslegern des Fahrgestells 1 liegt und ganz nach unten gefahren ist.

## Patentansprüche

1. Fahrbares Röntgengerät, bei dem auf einem Fahrgestell (1) angeordnet sind:
Ein Bedienpult (2), ein Stativ (3) und ein am Stativ (3) gelagerter Röntgenstrahler (5), wobei das Stativ (3) mit dem Röntgenstrahler (5) relativ zu dem Fahrgestell um eine vertikale Achse (6) schwenkbar ist, **dadurch gekennzeichnet,** daß das Bedienpult (2) und das Stativ (3) auf einer Montagegrundplatte (7) befestigt sind, welche mit dem Fahrgestell (1) um eine vertikale Achse drehbar verbunden ist, derart, daß unabhängig vom jeweiligen Schwenkbereich des Röntgenstrahlers (5) immer etwa gleiche Gewichtsverteilungen herrschen.

## Claims

1. Mobile X-ray apparatus, in which there are arranged on a wheeled carriage (1):
a control panel (2), a stand (3) and an X-ray source (5) mounted on the stand (3), wherein the stand (3) can be pivoted with the X-ray source (5) around a vertical axis (6) in relation to the wheeled carriage, **characterised in that** the control panel (2) and the stand (3) are secured to an assembly base plate (7) which is connected to the wheeled carriage (1) for rotation around a vertical axis, in such a way that approximately the same weight distributions always prevail, regardless of the pivoting range of the X-ray source (5) in each case.

## Revendications

1. Appareil à rayons X mobile, dans lequel sont montés sur un chariot (1) :
un pupitre (2) de commande, un statif (3) et un émetteur (5) de rayons X logé sur le statif (3), le statif (3) comportant l'émetteur (5) de rayons X pouvant basculer par rapport au chariot autour d'un axe vertical (6), caractérisé en ce que le pupitre (2) de commande et le statif (3) sont fixés à un plateau (7) de base de montage, qui est relié au chariot (1) de manière à pouvoir tourner autour d'un axe vertical, de telle sorte qu'il y ait toujours à peu près les mêmes répartitions de poids indépendamment de la zone de basculement de l'émetteur (5) de rayons X.
